# EUROPEAN PATENT APPLICATION

(11) **EP 4 368 231 A1**
(43) Date of publication of application: **15.05.2024**
(21) Application number: 22206363.8
(22) Date of filing: 09.11.2022
(51) Int. Cl.: A61M 25/01, A61M 27/00, A61M 25/00

(54) **INTERMITTENT URINARY CATHETER ASSEMBLY WITH PROTECTIVE SLEEVE ARRANGEMENT**

(71) Applicant: Wellspect AB, 431 21 Mölndal (SE)
(72) Inventor: ERIKSSON, Karsten, 475 45 Fotö (SE); LOGANATHAN, Sujithguru, 421 49 Västra Frölunda (SE); SANDBERG, Michael, 436 39 Askim (SE); CARLANDER, Rolf, 423 63 Torslanda (SE)
(74) Representative: Lind Edlund Kenamets Intellectual Property AB

(57) **Abstract**

An intermittent urinary catheter assembly comprises a catheter (1) having a proximal insertion end and a distal end opposite to the proximal insertion end. The catheter has a lumen extending from a drainage opening at or near the proximal insertion end to a discharge opening at or near the distal end. Further, a protective sleeve (2) arrangement is provided, having a proximal holder part (22), a distal holder part (21) and a compactable sleeve (23) connected to the proximal holder part and the distal holder part. The compactable sleeve has a compacted storage configuration and is extendable into an expanded configuration. The distal holder part is connected to the catheter at or near the distal end, and the proximal holder part and the distal holder part are releasably connected to each other.

## Description

### TECHNICAL FIELD OF THE INVENTION

The present invention relates to an intermittent urinary catheter assembly comprising a protective sleeve arrangement, the protective sleeve being compactable, and arrangeable in a compacted storage configuration and being extendable into an expanded configuration.

### BACKGROUND OF THE INVENTION

Urinary catheters are widely used for intermittent catheterization. In intermittent catheterization, the urinary catheter is inserted into the bladder, urine is discharged, and the catheter is then immediately removed. The whole procedure takes only a few minutes, and normally has to be repeated a number of times per day. In order to reduce the risk of damage to the urethral wall, the catheters are typically coated with a hydrophilic coating imparting an extremely low friction on the surface of the catheters. This coating is normally activated by applying a fluid medium (for example tap water or sterilized water) to the coating. The coating may be maintained activated during storage, providing a ready to use assembly, or be activated immediately prior to use, e.g. by release of a fluid from a separate compartment inside the package.

It is quite common that users of catheters experience Urinary Tract Infections (UTI). To reduce risk of infection, the urinary catheter as well as the fluid medium may be sterilized. Furthermore, the surface of the urinary catheter should preferably remain uncontaminated prior to use.

To avoid contamination, it is known to provide insertion aids, protective sleeves, etc, allowing the catheter to be handled and manipulated during preparation and insertion without having to directly touch the insertable part of the catheter. For example, US 9884167 by the same applicant discloses a tubular insertion aid arranged over the connector part of the catheter during storage. Upon use, the insertion aid may be released, and can be moved up and down over the catheter shaft for manipulation of the catheter in a no-touch manner. Another example is disclosed in WO 2010/006620. Here, a sleeve is arranged connected to the connector part of the catheter. During storage, the sleeve is arranged in a compacted state, but as soon as the package is opened and the catheter is extracted, the sleeve is unfolded into an expanded state, thereby covering all or most of the catheter shaft. With both these known arrangements, the user may hold the urinary catheter without it slipping due to the coating and without contaminating the sterilized surface.

However, known devices with protective sleeves and insertion aids are relatively complicated and costly to produce, and may also be cumbersome and difficult to use, in particular for users having poor dexterity and impaired hand movement. There is therefore still a need for improved protective measures that could provide the same or improved protection against contamination, which can be produced easier and more cost-efficiently, and/or which are easier and more intuitive to use, and with less need for hand movement control.

### SUMMARY OF THE INVENTION

It is therefore an object of the present invention to address the above-discussed needs, and to overcome or alleviate all, or at least some, of the above-discussed problems. Specifically, it is an object of the present invention to provide an improved intermittent urinary catheter assembly that could provide the same or improved protection against contamination, which can be produced easier and more cost-efficiently, and/or which can be easier and more intuitive to use, and with less need for hand movement control.

This object is obtained by an intermittent urinary catheter assembly as defined in the appended claims.

According to an aspect of the present invention, there is provided an intermittent urinary catheter assembly, comprising:
a catheter having a proximal insertion end and a distal end opposite to the proximal insertion end, the catheter having a lumen extending from a drainage opening at or near the proximal insertion end to a discharge opening at or near the distal end; and
a protective sleeve arrangement having a proximal holder part, a distal holder part and a compactable sleeve connected to the proximal holder part and the distal holder part, the compactable sleeve having a compacted storage configuration and being extendable into an expanded configuration,
wherein the distal holder part is connected to the catheter at or near the distal end, and the proximal holder part and the distal holder part are releasably connected to each other.

Since the distal holder part and the proximal holder part are releasably connected to each other, the sleeve will be maintained in its compacted storage position during storage, and also during use of the catheter, as long as the distal and proximal holder parts are not actively separated. This of great advantage for many reasons. First of all, it ensures that the catheter shaft is accessible for example for wetting of a hydrophilic surface thereon when arranged in the package. Further, the sleeve is not expanded into the expanded configuration until the user wishes this to happen, and actively perform the measure of separating the distal and proximal holder parts. For example, the user may wish to activate a hydrophilic surface after extraction of the catheter from the package, to visually inspect the catheter shaft prior to insertion, etc, and may for that reason want to completely or partly remove the catheter from the package prior to expansion of the sleeve. Some users may also want to handle the catheter without use of the sleeve, and for such users, the sleeve may be maintained in the compacted storage configuration during the entire catheterization procedure.

The protective sleeve arrangement is used to cover the surface of the catheter prior to use so that a user can use this for holding the catheter. Thereby, handling of the catheter is facilitated and contamination of the surface is prevented. This also reduces the risk of UTIs.

Still further, the releasably connected proximal and distal holder parts may also be reconnected to each other if the user would want to bring the sleeve back to its compacted storage position at a later stage. The proximal and distal holder parts may be reconnectable by the same connection arrangement as used initially, or through a different connection arrangement. However, non-reconnectable proximal and distal holder parts may also be provided.

At the same time, expansion of the sleeve is very simple and intuitive. For example, the user may simply just hold the proximal holder part, e.g. through a package in which the catheter is arranged, when the catheter should be removed from the package. The user may e.g. extract the catheter by grasping and pulling the connector end of the catheter. By simultaneously holding the proximal holder part, this will automatically also release the proximal holder part from the distal holder part, whereby the sleeve will be expanded.

Thus, handling of the catheter and the protective sleeve arrangement is simple and intuitive, also for users having poor dexterity, limited hand movement control, etc.

The protective sleeve arrangement can also be produced as a separate part, with the proximal and distal parts being connected together and the sleeve arranged in the compacted storage configuration. This can then easily and cost-efficiently be assembled on the catheter. For example, the connection can be obtained by a snap lock arrangement, such as a snap-fit, whereby the connection is obtained automatically when the protective sleeve arrangement is pushed into place during assembling.

Since the protective sleeve arrangement can be produced separately, it also allows the catheter to be produced in a conventional way, making the production cost-efficient and simple, and without any risk of e.g. damaging a hydrophilic coating or the like provided on the catheter shaft. It also makes it possible to produce the catheter and the protective sleeve arrangement at one location, or at different locations, and to have the assembling optionally taking place at another location.

In the present application, the term "proximal" is used to indicate the end or portion of a catheter that is inserted into the body of the user, i.e. the end or portion of the catheter shaft that during use is closer in proximity to the user's body and/or initially enters the user's body upon insertion. The term "distal" is used to refer to an end or portion of the catheter that is opposite the proximal end or portion and is typically further away from the user's body. For the sake of consistency, when the terms "distal" and "proximal" are used in the context of other components, such as the protective sleeve arrangement and the parts thereof, which are not intended for introduction into the user's body. For such other components, "proximal" refers to the end or portion that is closer to the proximal end of the catheter within the assembly, while "distal" refers to an end or portion located opposite to such proximal end or portion.

The distal holder part is preferably arranged with a continuous wall extending around the circumference of the catheter, and can e.g. be realized as a short tube. The inner diameter of the distal holder part is preferably corresponding to an outer diameter of a proximal end of a funnel or connector part of the catheter.

The proximal holder part is preferably also arranged with a continuous wall extending around the circumference of the catheter, and can e.g. also be realized as a short tube. The inner diameter of the proximal holder part is preferably similar to, or slightly greater than, the outer diameter of the catheter shaft.

The distal holder part and the proximal holder part are both preferably made of a more rigid material than the sleeve. Additionally, or alternatively, the proximal holder part and the distal holder part may both have a greater wall thickness than the sleeve. The compactable sleeve is preferably made of a flexible, collapsible material. The relatively thicker and/or more rigid proximal holder part also provides a stability to the protective sleeve arrangement also in the expanded configuration.

In an embodiment, the proximal holder part may be relatively stiff and rigid, making it difficult or impossible to compress to grasp the catheter shaft. In such embodiments, grasping of the catheter shaft may occur through the more flexible sleeve. However, in other embodiments, the proximal holder part may also be somewhat flexible, but still at least somewhat more rigid than the sleeve, thereby allowing the user to grasp the catheter shaft also via the proximal holder part. The user may e.g. compress the proximal holder part by use of the thumb and one or both of the index finger and the middle finger. By application of a moderate finger pressure, the catheter shaft can hereby be grasped and manipulated.

The catheter preferably comprises a funnel connected to or integrated with the distal end of a catheter shaft, the funnel defining the discharge opening, wherein the distal holder part is connected to the funnel. The funnel may also be referred to as a connector.

The compactable sleeve is preferably extendable towards the proximal insertion end, and wherein the compactable sleeve, in its expanded configuration, is arranged to cover a substantial part of the catheter, and preferably more than 50% of the length of a catheter shaft, and more preferably at least 70% of the length of the catheter shaft, and most preferably at least 90% of the length of the catheter shaft.

The compactable sleeve may be folded in the compacted storage configuration, and preferably with concertina or accordion folds, to form an accordion or concertina pleated configuration. Hereby, the sleeve may be folded in the storage configuration, and in the expanded use configuration, the folds are straightened. However, other ways of compacting the compactable sleeve are also feasible, such as rolling the sleeve material, or just lumping the material together.

The compactable sleeve may, in its extended configuration, have a length similar to the length of the catheter shaft. In embodiments, the compactable sleeve may have a length allowing it to completely cover the catheter shaft in the extended configuration. However, alternatively, the compactable sleeve may have a length shorter than the catheter shaft, thereby leaving a, preferably short, part of the catheter shaft toward the insertable proximal end exposed and not covered by the protective sleeve arrangement also when in the expanded configuration.

The compactable sleeve may be extendable to a length of between L-5 to L+5 cm, where L is the length of the catheter shaft. For a female catheter, the length may be between about 50 mm to 100 mm, and for a male catheter the length may be between about 300 mm and 430 mm. The length of the compactable sleeve in the extended state should preferably be long enough to minimize the risk of contamination of any part of the outer surface when touched upon. Generally, the larger the part of the surface that is covered by the protective sleeve, the lesser the risk of contamination if the surface accidentally comes into contact with an object, for example a sink or a towel in the near vicinity of the user.

The protective sleeve arrangement may, in the compacted storage configuration, have an axial length in the range of 10-60 mm, and preferably 15-50 mm, and more preferably 20-40 mm, such as approximately 20, 30 or 40 mm. Preferably, the axial length of the protective sleeve arrangement when in the compacted configuration is sufficiently low so that it does not extend over a coated part of the urinary catheter. More preferably, the axial length of the protective sleeve arrangement when in the compacted configuration is less than an uncoated part of the urinary catheter. Hereby, wetting of the entire coated part of the catheter shaft is facilitated.

The largest width of the compactable sleeve is preferably between 1 and 15 mm larger than the diameter of the urinary catheter. The lower limit leaves enough room surrounding the swelled coating of the catheter, so that the coating is not disturbed by the protective sleeve. The catheter, including the protective sleeve, should, however, not take up too much space. Therefore, oversizing the protective sleeve with respect to the catheter should be limited. Furthermore, the urinary catheter may be difficult to hold if the protective sleeve is too big, particularly if the catheter is slippery due to the provision of a coating.

The material of the compactable sleeve may be plastic foil such as polyethylene (PE) or polypropylene (PP) or coated or non-coated paper. However, other materials are also feasible. The protective sleeve is mainly used to cover the surface of the intermittent urinary catheter immediately prior to use. Thereby the protective sleeve need not be long-term impermeable. However, the protective sleeve should preferably not be too permeable because moisture and contamination from the hand should be prevented from permeating through the protective sleeve. However, there is no need for a complete impermeability, because it is only used while the urinary catheter is inserted, which normally only takes a few minutes. This means that many types of material are suited for the purpose, because the only requirement is that the material has to be foldable or in other ways compactable into a compressed state and from there be extendable with relative ease.

The distal holder part is preferably connected to the catheter by a snap lock attachment. This greatly facilitates the assembling process, since a snap fit or snap joint may hereby be obtained automatically as soon as the protective sleeve arrangement is brought into position on the catheter. The connection may be releasable or fixed.

The distal holder part and the proximal holder part are preferably connected to each other by a snap lock and/or press lock arrangement. This makes manufacturing simple and cost-efficient. Further, it also allows the distal holder part and proximal holder part to be reassembled and reconnected after having been separated in the expanded configuration.

The proximal holder part may comprise a protruding part, and preferably a flange, extending radially outwardly, and preferably at a proximal end of the proximal holder part. This protruding part facilitates manipulation of the protective sleeve arrangement. For example, it facilitates grasping of the proximal holder part through the material of a package enclosing the catheter, if a user would want to release the proximal holder part from the distal holder part simultaneously with extraction of the catheter, for automatic expansion of the sleeve. The protruding part also forms a stop preventing the user's fingers from accidentally slipping and sliding onto the exposed catheter shaft during handling.

The proximal holder part may be arranged to be inserted into the distal holder part. Thus, the releasable connection between the proximal and distal holder parts may hereby be provided between an outer surface of the proximal holder part and an inner surface of the distal holder part. The releasable connection may e.g. be formed as a snap joint or a friction joint. However, other types of joints are also feasible. Further, other embodiments, such as the distal holder part being arranged to be inserted into the proximal holder part, are also feasible.

The compressible sleeve may be connected to the proximal and distal holder parts in various ways. The sleeve may e.g. be attached to one or both of the holder parts by welding, gluing, by friction fit, or by attaching a heat shrinking foil around the sleeve.

The compactable sleeve may, in the compacted storage configuration, be primarily arranged over the proximal holder part. Additionally, or alternatively, the proximal holder part may have an axial length greater than the distal holder part. This is of advantage in particular if the proximal holder part is also to be used for indirect grasping of the catheter. It also provides a stability to the protective sleeve arrangement also in the expanded configuration.

However, in other embodiments, the compactable sleeve may, in the compacted storage, instead be arranged primarily over the distal holder part. In such embodiments, the distal holder part may have an axial length greater than the proximal holder part.

The compactable sleeve may, in the compacted storage configuration, be arranged outside the proximal and distal holder parts. Optionally, an outer tubular member may be provided to enclose and cover the compactable sleeve when in the compacted configuration.

Thus, in one embodiment the intermittent urinary catheter assembly may further comprise an outer sleeve arranged outside the compactable sleeve in the compacted storage configuration.

However, alternatively, the sleeve may be arranged inside the proximal and/or distal holder parts when in the compacted storage configuration.

The catheter shaft of the catheter may be provided with a hydrophilic outer surface, and preferably a hydrophilic coating.

The intermittent catheter assembly may further comprise a package, arranged to accommodate at least the insertable part of the catheter. In embodiment, the package may enclose and accommodate the entire catheter and the protective sleeve arrangement.

The package may also comprise a supply of wetting fluid, for wetting of the hydrophilic surface of the catheter. In an embodiment, the wetting fluid may be arranged in direct contact with the catheter, thereby maintaining the catheter in an activated, ready to use, state. Alternatively, the wetting fluid may be arranged in a separate compartment of the package, such as in a separate sachet arranged within the package. Hereby, the wetting fluid may be released at a suitable time prior to use, such as immediately before the intended use. Opening of the wetting fluid compartment for release of the wetting fluid can be made by e.g. applying a pressure on the wetting fluid compartment, such as by squeezing or pinching the sachet. However, other ways of opening the compartment are also feasible, as is per se well known in the art, such as by twisting, pulling, pushing, etc. According to yet another embodiment, the wetting fluid may be arranged in a separate compartment arranged in such a way that the catheter shaft can be moved through the compartment to be wetted. Such a compartment may e.g. be arranged so that the catheter shaft is pulled through the compartment upon extraction from a package.

The wetting fluid can e.g. be sterile water or saline. However, other fluids, such as vapor, may also be used.

The wetting fluid compartment may be provided as a separate part from the package, and either be loosely arranged in the package, or be connected to the package. Alternatively, the wetting fluid compartment may form an integrated part of the package.

In use, the user releases the wetting fluid to wet the catheter immediately prior to use, or moves the catheter shaft through the wetting fluid. Alternatively, the catheter is already wetted during storage, whereby no wetting action is necessary. The user then opens the package. The package may be provided with an opening at or in the vicinity of the distal end of the package, i.e. at a position close to where the distal end of the catheter is located when arranged in the package. The user may grip the connector of the catheter at the distal end and pull it out of the package. If the user wants to make use of the protective sleeve arrangement for handling of the catheter, he/she may at the same time hold the proximal holder part through the package, and thereby, the sleeve will during extraction of the catheter be pulled out over the catheter to the extended configuration where it covers all or part of the surface of the urinary catheter.

The package may be made of a foil, which is welded along the sides. For this purpose, materials like poly-ethylene-terephthalate (PETP), low-density poly-ethylene (LDPE), highdensity poly-ethylene (HDPE), poly-propylene (PP), poly-amide (PA), amorphous polyester (PET) and surface- treated paper may be used. However, other materials may also be used, as is per se well known in the art.

The urinary catheter is preferably maintained sterile when arranged in the package. Hereby, the catheter is completely sterile when it exits the package, thereby reducing the risk of getting infected when used.

Intermittent urinary catheters are per se well-known in the art. An example of a urinary catheter is a catheter provided as a tube that is made of polyurethane (PU), polyolefin, or other polymer(s), and which is provided with a funnel/connector in the distal end. In the opposite proximal end (the tip end), the catheter is provided with one or more eyelets that let urine enter into the tube. For a male catheter the insertable length may be 250-350 mm and for a female catheter the insertable length may be between 60-130 mm. The insertable length corresponds to the length of the tubular element except for a few centimeters that are closest to the connector. This part of the catheter is uncoated because the catheter is coated by dipping. Furthermore, the uncoated part enables welding or gluing of the connector or funnel to the catheter.

### BRIEF DESCRIPTION OF THE DRAWINGS

The present invention will be described in more detail with reference to the appended drawings, showing currently preferred embodiments of the invention.
Fig. 1 is a sideview of an intermittent urinary catheter assembly in accordance with an embodiment, where a protective sleeve arrangement is arranged in a compacted storage configuration.
Fig. 2 is a sideview on an intermittent urinary catheter assembly in accordance with an embodiment, where a protective sleeve arrangement is arranged in an expanded configuration.
Figs. 3a-3c are illustrations of an intermittent urinary catheter assembly in accordance with an embodiment, with the sleeve in a compacted state, where Figs. 3a and 3b are perspective views, with the compactable sleeve being removed for visibility, and Fig. 3c is a cross-sectional view.

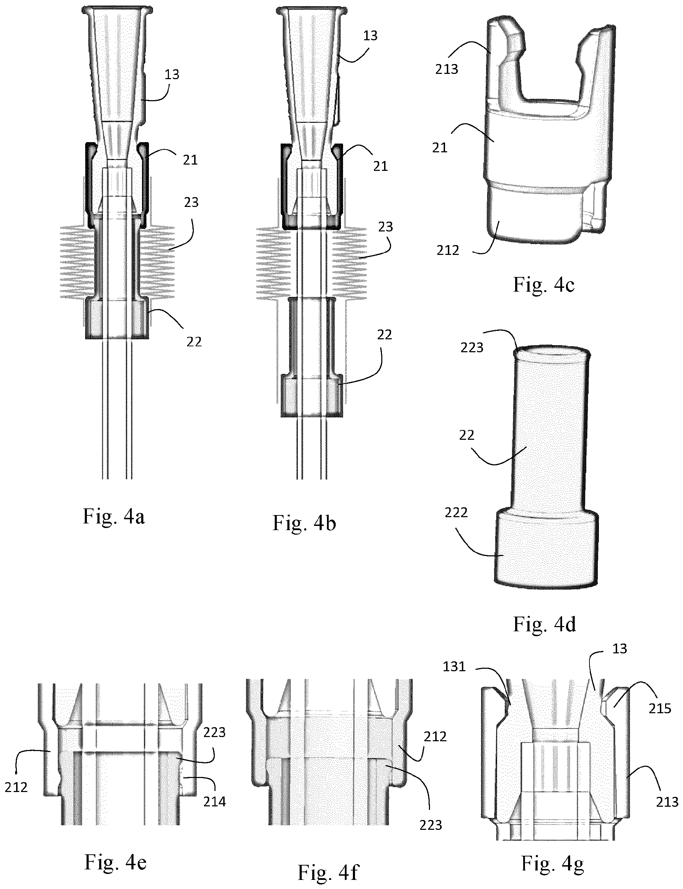

Figs. 4a-4g are illustrations of an intermittent urinary catheter assembly in accordance with another embodiment, where Figs. 4a and 4b are cross-sectional views showing the protective sleeve arrangement in a compacted configuration and a partly expanded configuration, respectively, Fig. 4c is a perspective view of the distal holder part, Fig. 4d is a perspective view of the proximal holder part, Figs. 4e and 4f are cross-sectional detailed views of the connection between the distal and proximal holder parts, and Fig. 4g is a cross-sectional detailed view of the connection between the distal holder part and the funnel of the catheter.
Figs. 5a-5d are illustrations of an intermittent urinary catheter assembly in accordance with another embodiment, where Figs. 5a and 5b are cross-sectional views showing the protective sleeve arrangement in a compacted configuration and a partly expanded configuration, respectively, Fig. 5c is a cross-sectional detailed view of the connection between the distal holder part and the funnel of the catheter and Fig. 5d is a cross-sectional detailed view of the connection between the distal and proximal holder parts.
Figs. 6a-6d are illustrations of a protective sleeve arrangement in accordance with another embodiment, where Fig. 6a is a perspective view from the side of the protective sleeve arrangement in a compacted configuration, Fig. 6b is a cross-sectional view of the same item as in Fig. 6a, Fig. 6c is a side-view of the same item as in Fig. 6a, and Fig. 6d is a perspective view from the side of the protective sleeve arrangement in an expanded configuration.
Fig. 7 is a cross-sectional view of an intermittent urinary catheter assembly in accordance with another embodiment.
Fig. 8 is a cross-sectional view of an intermittent urinary catheter assembly in accordance with still another embodiment.
Fig. 9 is a cross-sectional view of an intermittent urinary catheter assembly in accordance with yet another embodiment.
Fig. 10 is a perspective view from the side of an intermittent urinary catheter assembly in accordance with an embodiment, where the catheter has been arranged in a package.
Fig. 11 is an exploded view of the intermittent urinary catheter assembly of Fig. 10.

### DETAILED DESCRIPTION OF CURRENTLY PREFERRED EMBODIMENTS

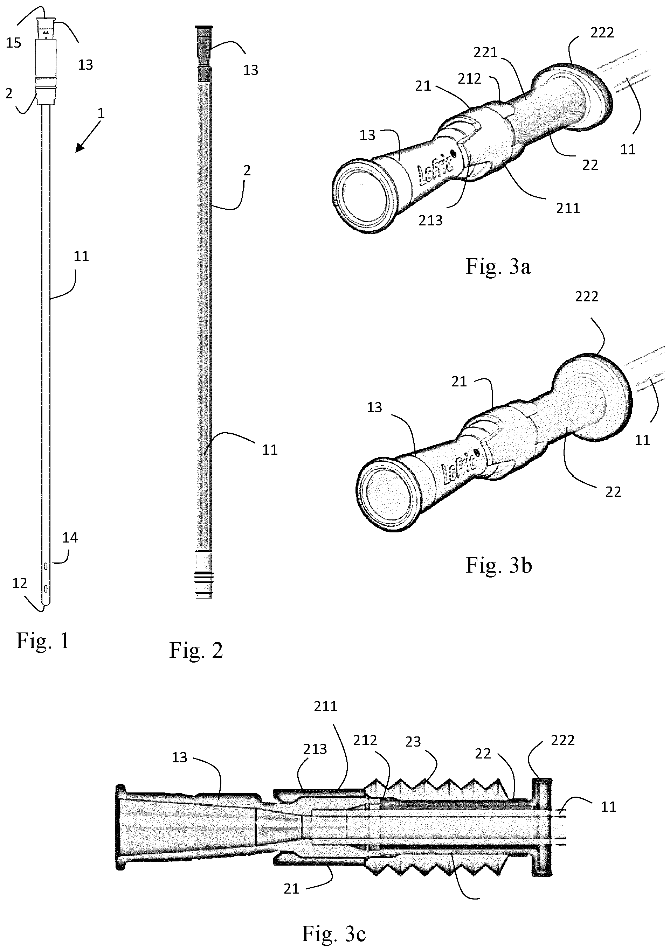

The intermittent urinary catheter assembly, as illustrated e.g. in Figs. 1 and 2, comprises a catheter 1 and a protective sleeve arrangement 2. The protective sleeve arrangement is shown in a compacted storage configuration in Fig. 1 and in an expanded use configuration in Fig. 2. Various embodiments of the protective sleeve arrangement will be discussed in more detail in the following.

The catheter comprises a catheter shaft 11, extending from a proximal end 12 and up to a connector or funnel 13, forming a non-insertable distal part of the catheter. An internal lumen extends through the catheter shaft, from inlet drainage openings, so-called eyelets 14 arranged at or in the vicinity of the proximal end 12, and up through the funnel 13, to end in discharge opening arranged at the distal end 15.

As discussed in the foregoing, a hydrophilic coating may be arranged on the catheter shaft, at least over an insertable length thereof. Alternatively, the catheter shaft may be formed by a hydrophilic material. The hydrophilic surface provides a very low friction to the catheter shaft when wetted.

The protective sleeve arrangement 2 is connected to the distal part of the catheter, and preferably connected to the funnel 13. In the compacted configuration, as illustrated in Fig. 1, the protective sleeve arrangement 2 only extends over a very limited distal part of the catheter. In the expanded configuration, as illustrated in Fig. 2, a compactable sleeve of the protective sleeve arrangement 2 has been expanded, so that the protective sleeve arrangement extends over and covers a significant part of the catheter shaft. In the illustrated embodiment, the protective sleeve arrangement 2 has a length such that it essentially covers the entire catheter shaft. However, a longer protective sleeve arrangement could also be used, having a length exceeding the catheter shaft. Alternatively, the protective sleeve arrangement may have a shorter length, so that it does not cover the full length of the catheter shaft, thereby leaving a part of the catheter shaft in the vicinity of the proximal end 12 uncovered and exposed also when the protective sleeve arrangement is in its expanded configuration.

Different embodiments of the protective sleeve arrangement will now be discussed in more detail.

In one embodiment, as illustrated in Figs. 3a-3c, the protective sleeve arrangement comprises a distal holder part 21, a proximal holder part 22 and a compactable sleeve 23.

The distal holder part 21 is connected to the catheter, and here to the proximal part of the funnel 13. The distal holder part in this embodiment comprises a tubular intermediate section 211, forming a continuous wall surrounding the catheter. At the distal end of the tubular intermediate section 211, one or more snap lock arms 213 may be provided. In the illustrative example, two such snap lock arms 213 are provided, arranged on opposite circumferential sides of the tubular intermediate section 211. The snap lock arms 213 may have small protrusions at the free ends, extending inwardly, towards the funnel 13. The funnel may be provided with one or more corresponding indentations, such as a waist formed in the vicinity of the proximal end of the funnel. Hereby, the snap lock arms 213 may form a snap joint or snap fit with the funnel when the distal holder part 21 is pushed up over the funnel 13.

Similar snap lock arms 212 may be arranged on the proximal side of the tubular intermediate section 211.

The proximal holder part 22 may comprise a generally cylindrical tubular section 221. At or in the vicinity of the proximal end of the proximal holder part 22, an outward protrusion, here in the form of an outwardly protruding flange 222, may be arranged. The outward protrusion 222 facilitates holding of the proximal holder part, e.g. through the walls of a package, and may also reduce the risk of slipping and sliding past the proximal end of the proximal holder part 22 during use. The flange may e.g. have an oval shape, as illustrated in Fig. 3a, or a circular shape, as illustrated in Fig. 3b. However, other shapes are also feasible.

At or adjacent the distal end, the tubular section 221 may be provided with an outward protrusion 223, such as an outwardly protruding ring, better seen in Fig. 4d. The outward protrusion 223 may interact with the proximal snap lock arms 213 of the distal holder part, to form a snap joint or snap fit between the proximal holder part and the distal holder part in the compacted configuration. This snap lock should be releasable. Preferably, the connection formed between the proximal and distal holder parts is much looser, and much easier to release, than the connection formed between the distal holder part and the catheter. This latter connection may even be a fixed, non-releasable joint.

The compactable sleeve 23 is connected with a distal end or part to the distal holder part 21, and with a proximal end or part to the proximal holder part 22. The connection can be provided by welding, shrink fit or the like.

The sleeve 23 is preferably made of a thin, flexible plastic material. The distal and proximal holder parts 21, 22 are preferably made of a thicker, more rigid material, and may e.g. be formed by injection molding.

A similar embodiment is illustrated in Figs. 4a-g. Here, Fig. 4a illustrates the protective sleeve arrangement in the compacted configuration, where the proximal holder part 22 and the distal holder part 21 are releasably connected to each other, and where the compactable sleeve 23 is in a compacted state. Fig. 4b illustrates the protective sleeve arrangement in a partly expanded configuration, where the proximal holder part 22 and the distal holder part 21 have been released from each other, and where the compactable sleeve 23 is in somewhat expanded state.

Figs. 4e and 4f illustrate the releasable connection between the proximal and distal holder parts in more detail. In the embodiment of Fig. 4e, the outward protrusion(s) 223 at the distal end of the proximal holder part interacts with inward protrusions 214 arranged at the proximal end of the distal holder part, in the same way as discussed in foregoing. Alternatively, as illustrated in Fig. 4f, one of the protrusions 214 and 223 have been removed. In the illustrative example, the inward protrusions 214 have been removed, thereby instead forming a straight, cylindrical wall in the distal part of the distal holder part. Hereby, a friction fit connection is obtained, instead of the snap joint of the embodiment of Fig. 4e, thereby forming an even looser connection.

Fig. 4g illustrate the connection between the distal holder part and the funnel 13 in more detail and illustrates how the inward protrusions 215 on the arms 213 interacts with the waist 131 formed in the funnel 13, thereby forming a snap joint.

In the embodiments discussed in relation to Figs. 3a-c and 4a-g, the proximal holder part 22 is long enough to accommodate most of the compactable sleeve 23, when in the compacted configuration. In particular, most of the compactable sleeve 23 is arrange overlying the cylindrical tubular section 221 of the proximal holder part 22.

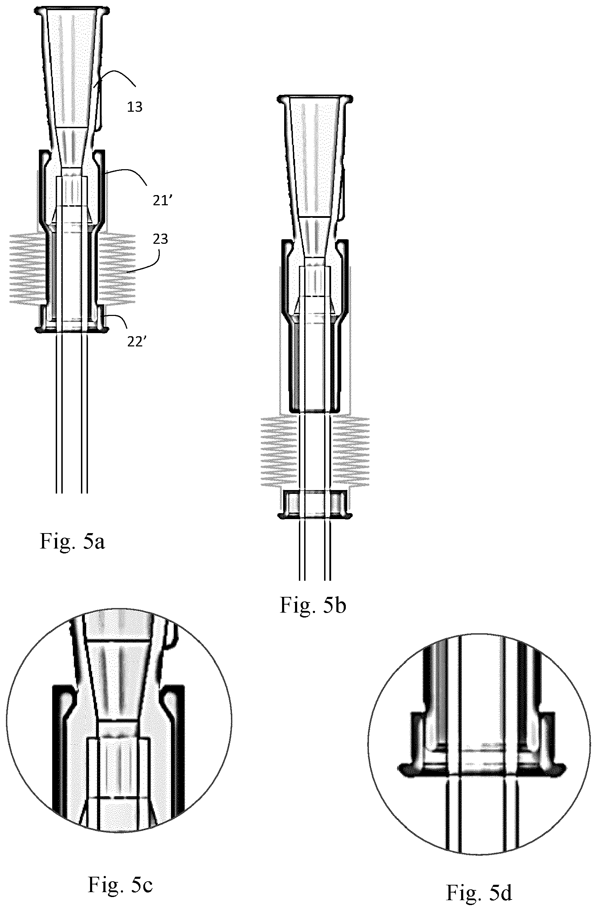

However, alternatively, the proximal holder part 22' may be shorter, and the distal holder part 21' be longer, such as illustrated in Figs. 5a-d. Hereby, the compactable sleeve 23 may primarily be arranged overlying the proximal part of the distal holder part 21' instead.

The connection between the distal holder part 21' and the funnel 13 may be provided in the same way as in the previously discussed embodiments, and as illustrated in Fig. 5c.

In the previously discussed embodiments, the distal end of the proximal holder part 22 has an inner diameter so that it can be inserted into the proximal end of the distal holder part 21, to form the releasable connection. However, the opposite arrangement is also feasible, as shown in the embodiment of Figs. 5a-d, and as illustrated in most detail in Fig. 5d. Here, the proximal end of the distal holder part 21' is insertable into the distal end of the proximal holder part 22', to form a releasable friction fit connection.

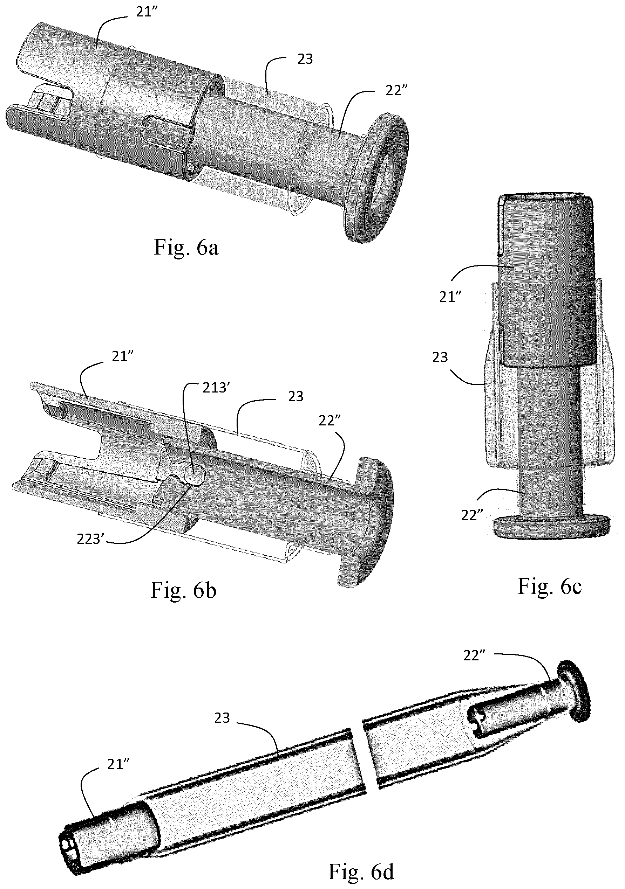

Another form of releasable connection between the distal holder part 21" and the proximal holder part 22" will now be discussed with reference to Figs. 6a-c. Here, the connection is instead formed by a an inwardly protruding pin or knob 213', extending inwardly from an inner surface of the distal holder part 21". The proximal holder part 22" is provided with an indentation or cut-out 223', extending generally in an axial direction from a distal end of the proximal holder part 22". The cut-out 223' may be provided with inward protrusions close to the opening. The cut-out 223' and the inward protrusion 213' form a snap lock arrangement, to form a releasable snap joint between the distal and proximal holder parts 21", 22".

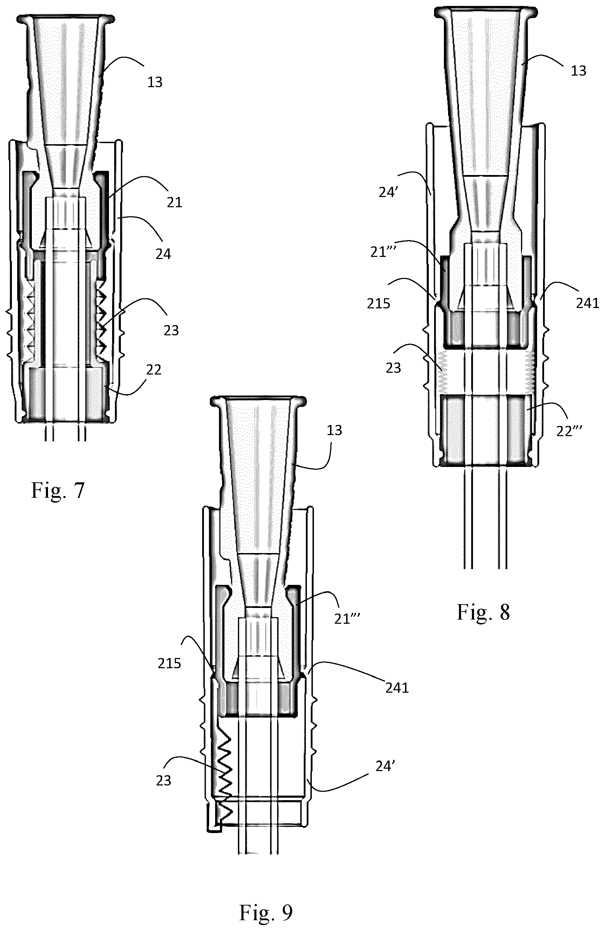

In the so far discussed embodiments, the sleeve is arranged outside the proximal and distal holder parts. However, it is also possible to provide a cover over the sleeve, for protective or esthetic reasons. Such embodiments will now be discussed with reference to Figs. 7-9.

In the embodiment of Fig. 7, the upper and lower holder parts 21, 22 are generally of the same type as discussed e.g. in relation to the embodiment of Figs. 4a-g. However, in addition to this, an outer sleeve 24 has been connected to the proximal holder part 22. Preferably, a proximal end or part of the outer sleeve 24 is connected to the proximal end or part of the proximal holder part 22. The outer sleeve 24 extends up over the compactable sleeve 23 when in the compacted configuration.

The embodiment of Fig. 8 is similar to the one discussed in relation to Fig. 7. Here, however, the outer sleeve 24' is provided with inward protrusions 241, such as an inwardly protruding ring, arranged to interact with outward protrusions 215 on the distal holder part 21"', to form a snap joint between these parts. This connection between the distal and proximal holder parts may be used in addition to the previously discussed releasable connections. However, alternatively, this snap joint may replace the other joints and connections, such as in the illustrative example. In this embodiment, the proximal holder part 22‴ can be made shorter, and need not be in any contact with the distal holder part 21‴. Instead, the outer sleeve 24' here forms an extension of the proximal holder part 22‴.

A similar embodiment is illustrated in Fig. 9, but here the outer sleeve 24' has been used to entirely replace the previously discussed proximal holder part, and instead in itself forms the proximal holder part. The compactable sleeve 23 may then be connected directly to the outer sleeve 24', for example at or adjacent the proximal end thereof.

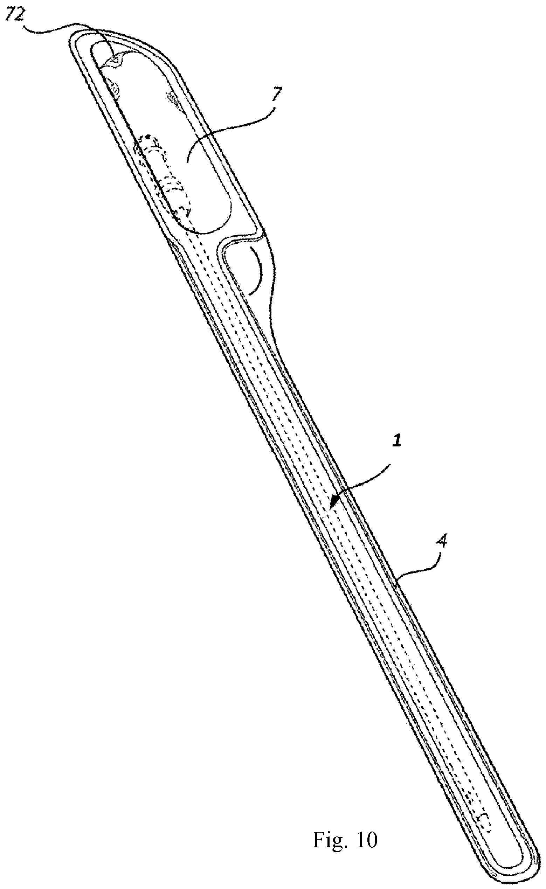

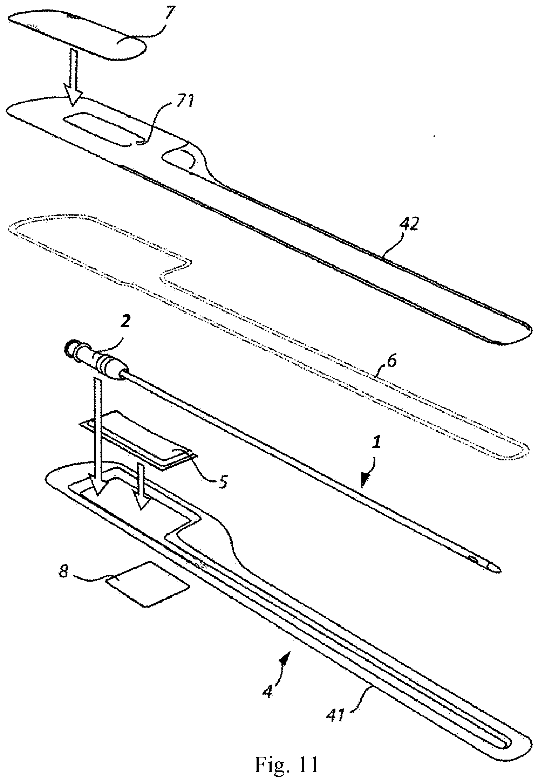

The above-discussed intermittent urinary catheter assembly, comprising a catheter and a protective sleeve arrangement, may further comprise a package 4 enclosing said catheter set. Such a catheter assembly will now be disclosed with reference to Figs. 10 and 11. The package may be structured and constituted in the same way as disclosed in US 2015/141966, said document hereby being incorporated in its entirety by reference.

The package 4 encloses the catheter 1, and a thereon assembled protective sleeve arrangement 2, as discussed previously. The package may further comprise a wetting fluid, preferably arranged in a wetting fluid container 5, for activation of the hydrophilic surface coating of the catheter.

The wetting fluid may be arranged separate from the catheter, in a wetting fluid container 5, such as a pouch or a sachet. The wetting fluid container is here openable by e.g. exerting a pressure to the container, whereby the wetting fluid is released into the package, thereby wetting the hydrophilic surface of the catheter. However, alternatively the wetting fluid may be arranged in direct contact with the catheter and the hydrophilic surface during storage, thereby providing an immediately ready-to-use catheter assembly, or be arranged in such a way that the catheter shaft is moved through the wetting fluid during extraction.

The wetting fluid is preferably an aqueous liquid, such as water or saline. Such wetting fluid containers and wetting fluids are per se well known in the art.

The package may be formed of sheet materials, such as a first sheet material 41 and a second sheet material 42, connected around the edges to form an inner cavity housing the catheter and the wetting fluid. The first and second sheet materials are preferably connected around the edges by means of welding, forming a welded edge joint 6. Preferably, the first and second sheet materials comprise laminated sheets, having a weldable inner layer and a protective outer layer. However, one or both of the sheets may alternatively be non-laminated, and be made of e.g. extruded or co-extruded material. The sheet materials are preferably of a flexible plastics material. For example, the sheets can be made of polymer materials such as polyethene, polypropylene, polyamide, and PET, or the receptacle can be made from a laminate of such polymer materials and/or aluminum, aluminum oxide, or oriented polypropylene (OPP).

One of the sheets, such as the sheet 41, may also be deep-drawn into a trough shape.

Further, the package is operable, for withdrawal of the catheter prior to an intended use. To this end, the package may be provided with tear openings, peel openings, or the like. For example, the sheets may be arranged to be peelable apart from each other at one or several locations. However, preferably the opening is arranged as a resealable opening. Such a resealable opening may e.g. be formed by forming a wholly or partially cut through nonclosed loop 71 in one of the sheets, or alternatively a closed loop, and to add a further opening sheet 7 on top of this loop. Attachment of the sheet 7 may e.g. be made by adhesive. The sheet 7 may comprise a non-adhered gripping tab 72 to facilitate opening. Additionally, the gripping tab may comprise a finger hole to facilitate opening even further. The tab may further be arranged at other positions than the one illustrated in the illustrative example.

In addition, there may be provided means for attaching the catheter package to a wall, a sink or the like. Such means may e.g. comprise an adhesive area arranged on one of the sheets, preferably the sheet not provided with the opening, and covered by a detachable cover sheet 8.

A method for producing a catheter assembly of this type includes the steps of:
- Separately producing the catheter and the protective sleeve arrangement.
- Assembling the protective sleeve arrangement on the catheter.
- Arranging the catheter assembly and a wetting fluid container on one of the sheets.

This sheet is preferably deep-drawn into a trough shape.
- Connecting the second sheet on top of the first sheet, thereby closing the package.
- Optionally, attaching the opening sheet and the cover sheet to the package.

Naturally, the order of these steps may be different, and e.g. the package may be partly closed prior to insertion of the catheter and/or the wetting fluid container. Further, attachment of the opening sheet and/or the cover sheet may be performed before or after any of the other steps.

The packed assembly may then e.g. be sterilized by radiation sterilization, or other sterilization methods.

In use, the following steps may be performed:
- If not already in a wetted, activated state, the wetting fluid is released to wet the hydrophilic surface of the catheter.
- The package is opened.
- The catheter is expelled from the package.
- The protective sleeve arrangement is brought from its compacted configuration to its expanded configuration, thereby covering all or at least a substantial part of the catheter shaft. The expansion of the protective sleeve arrangement can be made simultaneously with the removal of the catheter from the package. To this end, the user may hold the proximal holder part through the package when the catheter is pulled out, thereby simultaneously releasing the proximal holder part from the distal holder part. Alternatively, the catheter can first be removed from the package, and the protective sleeve arrangement be subsequently expanded.
- The protective sleeve arrangement may be used to grasp and manipulate the catheter for insertion of the catheter into the urethra. As the catheter is inserted deeper into the urethra, the protective sleeve arrangement will again be compacted, and will then remain in a compacted state, outside the urethra, during the drainage of urine from the bladder.
- After discharge of the urine, the catheter is removed from the urethra, and may be discarded. In this step, the protective sleeve arrangement may remain in a compacted configuration, or may again be expanded to the expanded configuration.

The present invention has now been disclosed with reference to specific exemplary embodiments. However, it will be acknowledged by the skilled addressee that several modifications are possible. For example, the proximal and distal holder parts may be differently shaped and structured, and the releasable connection between them may be realized also in other ways than the ones discussed in the foregoing. Further, the connection between the distal holder part and the catheter can be obtained in other ways. For example, the connection may instead be obtained by welding, gluing, shrink fit, etc. Further, the distal holder part can, as discussed in the foregoing, be connected to a funnel part of the catheter. However, it is also possible to connect the distal holder part to a distal part of the catheter shaft.

The above-discussed and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

## Claims

1. An intermittent urinary catheter assembly, comprising:
a catheter having a proximal insertion end and a distal end opposite to the proximal insertion end, the catheter having a lumen extending from a drainage opening at or near the proximal insertion end to a discharge opening at or near the distal end; and
a protective sleeve arrangement having a proximal holder part, a distal holder part and a compactable sleeve connected to the proximal holder part and the distal holder part, the compactable sleeve having a compacted storage configuration and being extendable into an expanded configuration,
wherein the distal holder part is connected to the catheter at or near the distal end, and the proximal holder part and the distal holder part are releasably connected to each other.

2. The intermittent urinary catheter assembly of claim 1, wherein the distal holder part and the proximal holder part are both made of a more rigid material than the sleeve.

3. The intermittent urinary catheter assembly of claim 1 or 2, wherein the compactable sleeve is made of a flexible, collapsible material.

4. The intermittent urinary catheter assembly of any one of the preceding claims, wherein the proximal holder part and the distal holder part both have a greater wall thickness than the sleeve.

5. The intermittent urinary catheter assembly of any one of the preceding claims, wherein the catheter comprises a funnel connected to or integrated with the distal end of a catheter shaft, said funnel defining the discharge opening, wherein the distal holder part is connected to the funnel.

6. The intermittent urinary catheter assembly of any one of the preceding claims, wherein the compactable sleeve is extendable towards the proximal insertion end, and wherein the compactable sleeve, in its expanded configuration, is arranged to cover a substantial part of the catheter, and preferably more than 50% of the length of a catheter shaft, and more preferably at least 70% of the length of the catheter shaft, and most preferably at least 90% of the length of the catheter shaft.

7. The intermittent urinary catheter assembly of any one of the preceding claims wherein the compactable sleeve is folded in the compacted storage configuration, and preferably with concertina or accordion folds, to form an accordion or concertina pleated configuration.

8. The intermittent urinary catheter assembly of any one of the preceding claims, wherein the compactable sleeve is extendable to a length of between L-5 to L+5 cm, where L is the length of the catheter shaft.

9. The intermittent urinary catheter assembly of any one of the preceding claims, wherein the distal holder part is connected to the catheter by a snap lock attachment.

10. The intermittent urinary catheter assembly of any one of the preceding claims, wherein the distal holder part and the proximal holder part are connected to each other by a snap lock and/or press lock arrangement.

11. The intermittent urinary catheter assembly of any one of the preceding claims, wherein the proximal holder part comprises a protruding part, and preferably a flange, extending radially outwardly, and preferably at a proximal end of the proximal holder part.

12. The intermittent urinary catheter assembly of any one of the preceding claims, wherein the proximal holder part is arranged to be inserted into the distal holder part.

13. The intermittent urinary catheter assembly of any one of the preceding claims, wherein the compactable sleeve, in the compacted storage configuration, is primarily arranged over the proximal holder part.

14. The intermittent urinary catheter assembly of any one of the preceding claims, wherein the proximal holder part has an axial length greater than the distal holder part.

15. The intermittent urinary catheter assembly of any one of the preceding claims, wherein the compactable sleeve, in the compacted storage configuration, is arranged outside the proximal and distal holder parts.

16. The intermittent urinary catheter assembly of any one of the preceding claims, further comprising an outer sleeve arranged outside the compactable sleeve in the compacted storage configuration.

17. The intermittent urinary catheter assembly of any one of the preceding claims, wherein a catheter shaft of the catheter is provided with a hydrophilic outer surface, and preferably a hydrophilic coating.
